(19) Europäisches Patentamt
European Patent Office
Office européen des brevets

(11) Veröffentlichungsnummer: **0 133 988**
**A2**

(12) # EUROPÄISCHE PATENTANMELDUNG

(21) Anmeldenummer: **84109100.2**

(22) Anmeldetag: **01.08.84**

(51) Int. Cl.[4]: **A 61 K 37/02**

(30) Priorität: **02.08.83 DE 3327856**
**05.10.83 DE 3336197**

(43) Veröffentlichungstag der Anmeldung: **13.03.85**
**Patentblatt 85/11**

(84) Benannte Vertragsstaaten: **AT BE CH DE FR GB IT LI LU NL SE**

(71) Anmelder: **HOECHST AKTIENGESELLSCHAFT,**
**Postfach 80 03 20, D-6230 Frankfurt am Main 80 (DE)**

(72) Erfinder: **König, Wolfgang, Dr., Eppsteiner Strasse 25,**
**D-6238 Hofheim am Taunus (DE)**
Erfinder: **Seidel, Heinz-Rüdiger, Dr., Im Kirschenfeld 15,**
**D-6370 Oberursel/Taunus (DE)**
Erfinder: **Sandow, Jürgen Kurt, Dr., Am Haideplacken 22,**
**D-6240 Königstein/Taunus (DE)**

(54) **Regulatorische Peptide enthaltende pharmazeutische Präparate mit protrahierter Freisetzung und Verfahren zu deren Herstellung.**

(57) Implantate enthaltend regulatorische Peptide oder deren Analoga als Wirkstoffe und natürliche Poly-D-(−)-3-Hydroxybuttersäure als biologisch abbaubaren Träger und Verfahren zu deren Herstellung werden beschrieben. Aus den Implantaten wird der Wirkstoff protrahierter freigesetzt.

EP 0 133 988 A2

ACTORUM AG

Regulatorische Peptide enthaltende pharmazeutische Präparate mit protrahierter Freisetzung und Verfahren zu deren Herstellung

Die Erfindung betrifft eine implantierbare Zubereitung von regulatorischen Peptiden und von deren Analoga mit protrahierter Freisetzung sowie Verfahren zur Herstellung der Implantate.

Es wurde schon beschrieben, daß aus Matrixtabletten enthaltend 7-Hydroxyethyltheophyllin als Wirkstoff und Poly-D(-)-3-Hydroxybuttersäure als biologisch abbaubares Trägermaterial der Wirkstoff in in vitro-Versuchen langsam abgegeben wird (Pharm. Ind. 45, S. 525-527 ((1983)).

Es wurde ferner beschrieben, daß aus Arzneimitteln enthaltend Peptide als Wirkstoffe und biodegradierbare Polymere als Trägersubstanzen die Peptide langsam freigesetzt Die Träger sind überwiegend synthetische Polyester aus Milchsäure und Copolymere aus Milch- und Glykolsäure (vgl. z.B. Europäische Patentanmeldungen mit den Veröffentlichungsnummern 0 052 510 und 0 058 481) sowie synthetische Aminosäurepolymere (vgl. US-Patentschrift 4 351 337). Der Nachteil synthetischer Polymere besteht darin, daß mit Polymerisationskatalysator-Rückständen zu rechnen ist. Solche Rückstände sind bei Arzneimitteln, und insbesondere bei Implantaten, unerwünscht.

Es wurde nun gefunden, daß sich natürliche Polyhydroxybuttersäure als Träger für peptidhaltige Implantate eignet, aus welchen der Wirkstoff protrahiert freigesetzt wird.

Die Erfindung betrifft daher Implantate enthaltend regulatorische Peptide oder deren Analoga als Wirkstoffe und natürliche Poly-D-(-)-3-Hydroxybuttersäure(PHB) der Formel

$$HO-\underset{\displaystyle CH_3}{CH}-CH_2-CO(O-\underset{\displaystyle CH_3}{CH}-CH_2-CO-)_nO-\underset{\displaystyle CH_3}{CH}-CH_2-COOH$$

worin n für eine Zahl zwischen 500 und 25000 steht, als biologisch abbaubaren Träger.

In den vorstehenden und folgenden Ausführungen steht "Peptide" für regulatorische Peptide und deren Analoga sowie deren physiologisch verträgliche Salze.

Die Erfindung betrifft ferner Verfahren zur Herstellung von Implantaten enthaltend regulatorische Peptide oder deren Analoga als Wirkstoffe, welche dadurch gekennzeichnet sind, daß man

1. den Wirkstoff in einem niedermolekularen Alkohol mit 1 bis 4 Kohlenstoffatomen, der gegebenenfalls mit bis zu 3 Fluoratomen substituiert ist, oder in Wasser oder in einem Gemisch dieser Lösungsmittel löst, mit der Poly-D-(-)-3-Hydroxybuttersäure vermischt, das feuchte Material trocknet und verpreßt oder

2. die Poly-D(-)-3-Hydroxybuttersäure in einem halogenierten aliphatischen $C_1$-$C_4$-Kohlenwasserstoff löst, mit einer Lösung des Wirkstoffs in einem niedermolekularen Alkohol mit 1 bis 4 Kohlenstoffatomen, der gegebenenfalls mit bis zu 3 Fluoratomen substituiert ist, vermischt, die erhaltene Lösung der Sprühtrocknung unterwirft und das getrocknete watteartige Material verpreßt, oder

3. die Poly-D(-)-3-Hydroxybuttersäure in einem halogenierten aliphatischen $C_1$-$C_4$-Kohlenwasserstoff löst, den Wirkstoff in dieser Lösung suspendiert, die Suspension auf eine geeignete Unterlage, z.B. in eine Glasschale gießt, das Lösungsmittel verdunstet und den entstandenen Film gegebenenfalls in Stücke geeigneter Größe teilt.

Die erhaltenen Preßlinge oder Filme lassen sich mahlen und durch Sieben in verschiedene Korngrößen aufteilen. Die festen Formkörper können als solche implantiert oder nach vorheriger Zerkleinerung in Form von Suspensionen injiziert werden.

Die regulatorischen Peptide (natürliche, synthetische und halbsynthetische), die auch als Salze eingesetzt werden können, sind in Wasser und niedermolekularen, gegebenenfalls mit Fluor substituierten, Alkohlen löslich. Als Alkohole kommen insbesondere Methanol und Trifluoräthanol in Betracht. Als Lösungsmittel für die PHB eignen sich insbesondere fluorierte und chlorierte Kohlenwasserstoffe, wie Methylenchlorid, Chloroform und 1,1,2-Trichlor-1,2,3-trifluor-äthan, wobei Methylenchlorid und Chloroform besonders geeignet sind.

Die PHB wird von Bakterien synthetisiert, wie z.B. von Alcaligenes eutrophus. Sie fällt in Form von kleinen Kügelchen in den Bakterien an und kann durch entsprechende Bedingungen in den Bakterien stark erhöht und daraus leicht isoliert werden (vgl. Pharma. Ind. 45, S. 525-527). Jeder Baustein der PHB besteht aus optisch reiner D-(-)-3-Hydroxybuttersäure.

Der biologische Abbau der PHB in vivo geht relativ langsam vor sich und trägt wenig zur Freisetzung eines Wirkstoffs aus einem Implantat bei. Die Freisetzung wird vor allem durch die Oberfläche des Implantats und die in ihm enthaltene Wirkstoffmenge gesteuert. Soll ein Peptid für längere Zeit in sehr geringen Mengen freigesetzt werden, so empfiehlt sich ein Implantat mit geringer Oberfläche und geringem Peptidgehalt, z.B. in Form von Preßlingen. Die Freigabe aus dem Preßling läßt sich weiter verringern, indem man das Implantat mit einem Überzug aus PHB oder anderen biologisch abbaubaren Polymeren wie Polymilchsäure oder Polymilchsäure/Polyglykolsäure-Copolymeren oder mit Polymeren wie Ethylcellulose, Poly(meth)acrylsäurederivaten oder Polydimethylsiloxanen ganz oder teilweise beschichtet.

Mit solchen Implantaten läßt sich eine im wesentlichen gleichmäßige Freisetzung von Peptiden bis zu einem Jahr erreichen. Sie lassen sich operativ leicht entfernen, falls die Behandlung abgebrochen werden sollte.

Während die nach Methode 1 hergestellten Tabletten zur Implantion von Anfang an eine relativ konstante Menge eines regulatorischen Peptids freigeben, setzen die nach Methode 2 erhaltenen Implantate in den ersten Tagen relativ viel Peptid frei und geben anschließend konstante kleine Mengen ab. Mit den erfindungsgemäßen Implantaten ist also eine gute Anpassung an den gewünschten Freigabeverlauf des Wirkstoffs möglich.

Diese langen Freisetzungsraten überraschen, wenn man mit den dagegen raschen Liberationsraten von etwa 40 Tagen mit den Copolymeren aus Milchsäure und Glykolsäure vergleicht (siehe Europäische Patentanmeldung mit der Veröffentlichungsnummer 0 058 481).

Für kürzere Liberationszeiten kommen als Implantate sehr kleine Tabletten oder andere kleine Formkörper in Frage, auf die die Gesamtdosis verteilt ist. Infolge der wesentlich größeren Oberfläche, die eine Vielzahl von Arzneistoffsträgern in Vergleich zu einem einzigen Formling besitzt, erfolgt die Freisetzung schneller. Bevorzugt sind kleine Partikel, wie sie durch Zerkleinern von Tabletten* hergestellt werden können. Sie können nach Suspension in einem geeigneten Medium injiziert werden. Hierbei sollte eine bestimmt Korngröße nicht überschritten werden; zweckmäßig liegen die Teilchengrößen im Bereich von 0,1 bis 200 µm.
*und Filmen

Zur Suspension und Injektion der Partikel kann phyiologische Kochsalzlösung verwendet werden, in der z.B. 1 % Hydroxypropylmethylcellulose(Methocel$^{R}$ E5) Carboxymethylcellulose(Blanose$^{R}$ 7 LF) oder Polyethylenglykol-Sorbitanmonostearat (Tween$^{R}$ 20) gelöst ist.

Regulatorische Peptide sind physiologisch wirksame endogene Peptide. Man bezeichnet sie auch als Peptidhormone, die man je nach Ort der Synthese oder Freisetzung in z.B. Peptidhormone des Hypothalamus, der Hypophyse, des Gastrointestinaltrakts oder der Schilddrüse einteilt. Diese Einteilung ist heute unzweckmäßig, da man weiß, daß die sog. Peptidhormone nicht nur an einer Stelle im Körper produziert werden und neben ihrer endokrinen Wirkungsweise auch parakrin oder neurokrin wirken können.

Eine Einteilung dieser Peptide nach Indikationen ist ebenfalls nicht zweckmäßig, da sie je nach Wirkungsort und Dosis die verschiedensten therapeutischen Aktivitäten entwickeln können.

Vertreter regulatorisher Peptide, die die erfindungsgemäßen Implantate enthalten können, sind z.B. Oxytocin, Vasopressin, Thyroliberin, das anorexigenische Peptid, Gonadoliberin, Calcitonin, Parathormon, der epiderme Wachstumsfaktor, Sekretin, das vasoaktive intestinale Peptid, Somatoliberin, das gastrininhibierende bzw. glukoseabhängige insulinotrope Peptid, Glukagon, das pankreatische spasmolytische Peptid, Somatostatin, Bombesin, das gastrinfreisetzende Peptid, Motilin, Neutrotensin, Substanz P, Sauvagin, Corticoliberin, Urotensin I und II, Angiotensin I und II, Bradykinin, Corticotropin, Enkephaline, Dynorphin, Dermophin, Casomorhine, Gastrin, Cholecystokinin, Cärulein, Thymusfaktoren, Interferone, Insulin, Wachstumshormon und Prolaktin.

Von besonderer Bedeutung sind die stark wirksamen Analoga des Gonadoliberins, wie z.B.

$[D\text{-}Ser(Bu^t)^6]$Gonadoliberin-(1-9)nonapeptid-äthylamid (Buserelin, Drugs of the Future 4, 1979, S. 175-177, 8, 1983, S. 254),

[D-Trp$^6$] Gonadoliberin (Drugs of the Future 3, 1978, S. 645-646),
[D-Trp$^6$] Gonadoliberin(1-9)-nonapeptid-äthylamid (Drugs of the Future 7, 1982, S. 637-642),
[D-Leu$^6$] Gonadoliberin(1-9)-nonapeptid-äthylamid (Drugs of the Future 7, 1982, S. 882-886),
[D-Ser(Bu$^t$)$^6$, AzaGly$^{10}$] Gonadoliberin (Drugs of the Future 5, 1980, S. 191-192; 8, 1983, S. 364-365),
[D-Trp$^6$, N-MeLeu$^7$] Gonadoliberin-(1-9)-nonapeptid-äthylamid (Drugs of the Future 8, 1983, S. 347-350),
[D-α-Aminodipinsäure-δ-tert.butylester$^6$] Gonadoliberin-(1-9)-nonapeptid-äthylamid (Deutsche Offenlegungsschrift 30 20 941),
[D-Lys(Boc)$^6$] Gonadoliberin(1-9)-nonapeptid-äthylamid (Deutsche Patentschrift 2 438 350),
[D-3-(2.4.6.-Trimethylphenyl)-Ala$^6$] Gonadoliberin und [D-3-(2-Napthyl)-Ala$^6$] Gonadoliberin (J. Med. Chem. 25, 1982, S. 795-801).

Diese Peptide reduzieren in hoher Dosierung die Plasma-Spiegel von Lutropin und Follitropin und damit die der gonadalen Steroide Testosteron und Östradiol. Diese Derivate lassen sich deshalb bei hormonabhängigen Tumoren, wie z.B. Prostata- oder Mamma-Carcinom, wie auch bei der Endometriose und der Pubertas praecox der Kinder anwenden. Für diese Therapie ist eine ständige gleichmäßige Freisetzung des Wirkstoffs besonders wichtig. Mit der erfindungsgemäßen Zubereitung kann mit einer einmaligen Applikation für Wochen oder Monate die notwenige Menge des Wirkstoffs freigesetzt werden, der sonst täglich 2-3 mal parenteral oder intranasal appliziert werden muß. Damit wird besonders die Anwendung bei älteren Personen und Kindern gegen Anwendungsfehler gesichert (Compliance).

Eine weitere wichtige Anwendung der erfindungsgemäßen Zubereitung ist die protrahierte Freisetzung von Somatostatin und Somatostatinanaloga, die überall dort eingesetzt werden kann, wo Somatostatininfusionen einen günstigen Effekt zeigen; z.B. bei Blutungen des gastrointestinalen Traktes, bei Magengeschwüren, bei der Behandlung von Tumoren, die durch Somatostatin hemmbare Hormone produzieren, wie z.B. beim Zollinger-Ellison-Syndrom, Verner-Morrison-Syndrom, oder bei Insulin- bzw. Glukagon-produzierenden Tumoren, bei hormonabhängigen Tumoren, wenn die entsprechenden Hormone durch Somatostatin hemmbar sind, bei gewissen Leukämiearten, bei Stoffwechselstörungen mit erhöhten durch Somatostatin hemmbaren Hormonspiegeln, wie z.B. der rheumatischen Arthritis, bei der Plasma-Insulin und -Wachstumshormon zu hoch sind, bei Akromegalie, Schuppenflechte, bei Diabetes mellitus (Hemmung des Glukagons), beim Chondrosarkom und bei Schockzuständen.

Hochwirksame Analoga des Somatostatins sind Verbindungen in denen z.B. $Trp^8$ durch D-Trp oder 5-F-D-Trp substituiert ist oder verkürzte cyclische Verbindungen, wie z.B.

Pro-Phe-D-Trp-Lys-Thr-Phe (cyclisch)

(Nature <u>292</u>, 1981, S. 55) oder

H-D-Phe-Cys-Phe-D-Trp-Lys-Thr-Cys-Thr-ol

(Life Sci. <u>31</u>, 1982, S. 1133-1140).

Auch die Therapie der oberen gastrointestinalen Blutungen mit Sekretininfusionen läßt sich durch die neue galenische Zubereitung vereinfachen.

Das Verhältnis von Wirkstoff zu Trägermaterial kann in weiten Grenzen variieren. Da die Peptide in geringen Dosierungen verabreicht werden, ist der Anteil des Trägermaterials in den Implantaten relativ hoch (z.B. 100 : 1 bis 10000 : 1).

Beispiel 1:

2,5 g PHB wurden mit einer methanolischen, 2,875 mg Buserelinacetat (entsprechend 2,5 mg Buserelin) enthaltenden Lösung befeuchtet und durchgemischt. Das feuchte Material wurde unter Schütteln im Vakuum getrocknet. Der Vorgang wurde mit reinem Methanol mehrmals wiederholt. Die trockene Mischung wurde zu 50 mg schweren Tabletten (Implantaten) mit einem Gehalt von 50 µg Buserelin verpreßt.

Beispiel 2:

Es wurden 2,875 mg Buserelinacetat (entsprechend 2,5 mg Buserelin) in 30 ml Methanol und 2,5 g PHB in 70 ml Chloroform gelöst. Beide Lösungen wurden vereinigt und einer Sprühtrocknung unterworfen. Man erhielt ein flockiges Pulver, aus dem Tabletten zu 50 mg mit einem Gehalt von 50 µg Buserelin gepreßt wurden.

Beispiel 3:

Die unter Beispiel 1 oder 2 hergestellten Preßlinge wurden mikronisiert. Die erhaltenen Partikel wurden durch Siebung in Korngrößenbereiche bis zu etwa 200 µm aufgeteilt. Die Fraktionen wurden zur Injektion in physiologischer Kochsalzlösung mit 1 % Carboxymethylcellulose in einer Konzentration von 50 mg/ml suspendiert.

Beispiel 4:

2,5 g PHB wurden in 25 g Chloroform gelöst. In dieser Lösung wurden 287,5 mg Buserelinacetat (entsprechend 250 mg Buserelin) suspendiert. Die Suspension wurde in eine Petrischale gegossen. Man ließ das Lösungsmittel langsam verdunsten. Es entstand ein Film, der in 1 $cm^2$ große Blättchen mit einem Gehalt von etwa 5 mg Buserelin zerteilt wurde.

Beispiel 5:

Biologische Prüfung der Zubereitungen an Ratten

Es wurden zwei gleich schwere und gleich große Implantationsmaterialien aus PHB und einem Copolymerisat aus Milchsäure und Glykolsäure (PLG), die analog Beispiel 1 hergestellt wurden, untersucht. Die Prüfung erfolgte an erwachsenen, 400 g schweren Ratten, unter Bestimmung der täglichen freigesetzten Peptidmenge durch pharmakokinetischen Nachweis mittels spezifischem Radioimmunoassay. Bei dem PHB-Implantat fand sich pro Tag eine Freisetzung von 0,203 ± 0,038 ng Buserelin. Dagegen zeigte das PLG-Implantat eine Freisetzung von 1,075 ± 0,029 ng Buserelin/Tag. Aus der kumulativen Freisetzungsrate wurde die Gesamtdauer der Peptidfreisetzung berechnet. Sie beträgt für das PHB-Implantat 221 ± 29 Tage, für das PLG-Implantat 46,5 ± 1,2 Tage. Damit ist das PHB-Implantatmaterial für eine langfristige Freisetzung von Peptiden wesentlich besser geeignet, als das zum Vergleich verwendete Co-Polymerisat aus Milchsäure und Glykolsäure (50:50).

PATENTANSPRÜCHE:

1. Implantat, enthaltend ein regulatorisches Peptid oder eines seiner Analoga als Wirkstoff und natürliche Poly-D(-)-3-Hydroxybuttersäure der Formel

$$HO-\underset{\underset{CH_3}{|}}{CH}-CH_2-CO-(O-\underset{\underset{CH_3}{|}}{CH}-CH_2-CO-)_n O-\underset{\underset{CH_3}{|}}{CH}-CH_2-COOH$$

worin n für eine Zahl zwischen 500 und 25000 steht, als biologisch abbaubaren Träger.

2. Implantat gemäß Anspruch 1, dadurch gekennzeichnet, daß es Buserelin(acetat) als Wirkstoff enthält.

3. Verfahren zur Herstellung von Implantaten gemäß Anspruch 1, dadurch gekennzeichnet, daß man

1. den Wirkstoff in einem niedermolekularen Alkohol mit 1 bis 4 Kohlenstoffatomen, der gegebenenfalls mit bis zu 3 Fluoratomen substituiert ist, oder in Wasser oder in einem Gemisch der beiden Lösungsmittel löst, mit der Poly-D(-)-3-Hydroxybuttersäure vermischt, das feuchte Material trocknet und verpreßt oder

2. die Poly-D(-)-3-Hydroxybuttersäure in einem halogenierten aliphatischen $C_1$-$C_4$-Kohlenwasserstoff löst, mit einer Lösung des Wirkstoffs in einem niedermolekularen Alkohol mit 1 bis 4 Kohlenstoffatomen, der gegebenenfalls mit bis zu 3 Fluoratomen substituiert ist, vermischt, die erhaltene Lösung der Sprühtrocknung unterwirft und das getrocknete Material verpreßt, oder

3. die Poly-D(-)-3-Hydroxybuttersäure in einem halogenierten aliphatischen $C_1$-$C_4$-Kohlenwasserstoff löst, den Wirkstoff in dieser Lösung suspendiert, die Suspension auf eine geeignete Unterlage gießt, das Lösungsmittel verdunstet und den entstandenen Film gegebenenfalls in Stücke geeigneter Größe teilt.

4. Verfahren gemäß Anspruch 3, dadurch gekennzeichnet, daß der Preßling oder Film in einem weiteren Schritt zerkleinert und in einem für Injektionszwecke geeigneten Lösungsmittel suspendiert wird.

5. Verfahren gemäß Anspruch 3, dadurch gekennzeichnet, daß man den Wirkstoff in Methanol löst.

6. Verfahren gemäß Anspruch 3, dadurch gekennzeichnet, daß man die Trägersubstanz in Chloroform löst.

Patentansprüche für Österreich:

1. Verfahren zur Herstellung von Implantaten enthaltend ein regulatorisches Peptid oder eines seiner Analoga als Wirkstoff und einen biologisch abbaubaren Träger, dadurch gekennzeichnet, daß man

   1) den Wirkstoff in einem niedermolekularen Alkohol mit 1 bis 4 Kohlenstoffatomen, der gegebenenfalls mit bis zu 3 Fluoratomen substituiert ist, oder in Wasser oder in einem Gemisch der beiden Lösungsmittel löst, mit natürlicher Poly-D(-)-3-Hydroxybuttersäure der Formel

   $$HO-\underset{CH_3}{CH}-CH_2-CO-(O-\underset{CH_3}{CH}-CH_2-CO-)_n O-\underset{CH_3}{CH}-CH_2-COOH$$

   worin n für eine Zahl zwischen 500 und 25000 steht vermischt, das feuchte Material trocknet und verpreßt,

   2) natürliche Poly-D(-)-3-Hydroxybuttersäure der Formel

   $$HO-\underset{CH_3}{CH}-CH_2-CO-(O-\underset{CH_3}{CH}-CH_2-CO-)_n O-\underset{CH_3}{CH}-CH_2-COOH$$

   worin n für eine Zahl zwischen 500 und 25000 steht in einem halogenierten aliphatischen $C_1$-$C_4$-Kohlenwasserstoff löst, mit einer Lösung des Wirkstoffs in einem niedermolekularen Alkohol mit 1 bis 4 Kohlenstoffatomen, der gegebenenfalls mit bis zu 3 Fluoratomen substituiert ist, vermischt, die erhaltene Lösung der Sprühtrocknung unterwirft und das getrocknete Material verpreßt, oder

   3) natürliche Poly-D(-)-3-Hydroxybuttersäure der Formel

   $$HO-\underset{CH_3}{CH}-CH_2-CO-(O-\underset{CH_3}{CH}-CH_2-CO-)_n O-\underset{CH_3}{CH}-CH_2-COOH$$

worin n für eine Zahl zwischen 500 und 25000 steht in einem halogenierten aliphatischen $C_1$-$C_4$-Kohlenwasserstoff löst, den Wirkstoff in dieser Lösung suspendiert, die Suspension auf eine geeignete Unterlage gießt, das Lösungsmittel verdunstet und den entstandenen Film gegebenenfalls in Stücke geeigneter Größe teilt.

2. Verfahren gemäß Anspruch 1, dadurch gekennzeichnet, daß man Buserelin(acetat) als Wirkstoff einsetzt.

3. Verfahren gemäß Anspruch 1, dadurch gekennzeichnet, daß der Preßling oder Film in einem weiteren Schritt zerkleinert und in einem für Injektionszwecke geeigneten Lösungsmittel suspendiert wird.

4. Verfahren gemäß Anspruch 1, dadurch gekennzeichnet, daß man den Wirkstoff in Methanol löst.

5. Verfahren gemäß Anspruch 1, dadurch gekennzeichnet, daß man die Trägersubstanz in Chloroform löst.